# EUROPÄISCHE PATENTSCHRIFT

(11) **EP 0 917 647 B1**
(45) Veröffentlichungstag und Bekanntmachung des Hinweises auf die Patenterteilung: **02.05.2003**
(21) Anmeldenummer: 97937559.9
(22) Anmeldetag: 06.08.1997
(51) Int. Cl.: G01N 17/00

(54) **VERFAHREN ZUR BESTIMMUNG VON EIGENSCHAFTSÄNDERUNGEN EINER PROBE**
METHOD OF DETERMINING VARIATIONS IN THE PROPERTIES OF A SAMPLE
PROCEDE POUR DETERMINER LES MODIFICATIONS DES PROPRIETES D'UN ECHANTILLON

(30) Priorität: 10.08.1996 DE 19632349
(43) Veröffentlichungstag der Anmeldung: 26.05.1999
(73) Patentinhaber: Kockott, Dieter Dr., 63456 Hanau (DE)
(72) Erfinder: Kockott, Dieter Dr., 63456 Hanau (DE)
(74) Vertreter: Stoffregen, Hans-Herbert, Dr. Dipl.-Phys.
(86) Internationale Anmeldenummer: EP9704294
(87) Internationale Veröffentlichungsnummer: WO98007017

(56) Entgegenhaltungen:
- DE-A- 3 443 604
- GB-A- 2 174 800
- US-A- 3 693 020

## Beschreibung

Die Erfindung bezieht sich auf ein Verfahren zur Bestimmung von Eigenschaftsänderungen einer einer künstlichen Bewitterung ausgesetzten Probe.

Verfahren zur Schnellbewitterung mit kommerziellen Bewitterungsgeräten sind zum Beispiel der DE 28 16 548 A1, EP 0 320 209 A2, DE-AS 1 904 097, US 4,760,748, US 4,874,952, DE 25 02 239 B2 oder DE 35 04 793 C2 zu entnehmen. Mit den bekannten Geräten erfolgt eine Simulation der Umgebungsbedingungen, wobei Bestrahlungsstärke und Temperatur auf das Maximum der jeweiligen Einsatzbedingungen eingestellt werden. Hierdurch wird eine Zeitraffung ermöglicht, die in Abhängigkeit vom Material bei Faktoren von 10 - 20 liegen kann. Aus der GB 21 74 800 A ist eine Vorrichtung bekannt, mit der die von einer Lampe reflektierte Strahlung kontinuierlich gemessen wird, um Farbveränderungen festzustellen. Die Probe selbst wird auf einem Metallblock angeordnet, der auf eine gewünschte Temperatur eingestellt wird.

Um eine schnelle Analyse von Kunststoffen zu ermöglichen, werden nach der DE 43 31 296 A1 ein Verfahren und eine Vorrichtung vorgeschlagen, bei der der zu bestimmende Kunststoff mit Laserlicht bestrahlt wird, um anschließend Ablations- bzw. Verdampfungsprodukte mit einem Fourier-Transform-Infrarotspektrometer zu identifizieren.

Um die Absorptions- und Laserfestigkeit von optischen Schichten zu ermitteln, werden nach der DE 41 09 469 A1 Proben auf einem Sensor angeordnet und durchstrahlt.

Um den Alterungszustand von Kunststoffgegenständen unabhängig von ihrem Alter zu ermitteln, wird nach der DE 31 21 928 A1 vorgeschlagen, die Kunststoffgesenstände mit einer Lichtquelle, die Licht im gesamten sichtbaren Spektralbereich aussendet, zu beleuchten und sodann die Remission an zumindest einer Stelle der Oberfläche der Kunststoffgegenstände zu ermitteln.

Um eine unzulässige Temperaturerhöhung bei der Bestimmung von Eigenschaftsänderungen von Proben aufgrund photochemischer Prozesse weitgehend auszuschließen, ist es zum Beispiel nach der DE 34 43 604 A1 bekannt, die Proben mit Licht im Wellenlängenbereich unter 400nm zu bestrahlen, um eine Erwärmung zu reduzieten. Ergänzend werden die Proben mit Luftströmen und Ventilatoren oder ähnlichem geköhlt. Mit dem bekannten Verfahren können sodann Farbunterschiede festgestellt werden, und zwar durch einen Vergleich vor und nach der Bestrahlung.

Der DE 40 02 985 A1 sind Maßnahmen zu entnehmen, um eine unerwünschte Erwärmung einer Probe während der Bestrahlung in einem der Sonnenstrahlung entsprechenden Spektralbereich mit einer Bestrahlungsstärke, die mehr als 30fach stärker als die der Sonnenstrahlung ist, zu vermeiden, indem ein Luftstrom über die Oberfläche der Probe geleitet wird.

Nach der DE 42 10 585 A1 wird aus einem gemessenen spektralen Absorptionsvermögen einer Probe und der Spektralverteilung der Sonnenstrahlung die absorbierte Strahlung berechnet, die zur Abschätzung des Alterungsverhaltens der Probe dient.

Aus der US 3,693,020 ist ein Verfahren der eingangs genannten Art zu entnehmen, bei dem durch integrierende Messgeräte in Form von Photozellen die eine Probe durchsetzende Strahlung gemessen wird, um Eigenschaftsänderungen einer Probe zu bestimmen.

Ungeachtet der Vielzahl der bekannten Verfahren und Vorrichtungen zur Schnellbewitterung von Gegenständen halten sich die erzielbaren Raffungsfaktoren in Grenzen, insbesondere dann, wenn vermieden werden soll, daß die Probe in einem Umfang erwärmt wird, daß neben photochemisch bedingten Alterungsprozessen andere temperaturabhängige Einflüsse auftreten. Ferner erfolgt eine Überprüfung der Proben grundsätzlich erst, nachdem die Behandlung erfolgt ist.

Der vorliegenden Erfindung liegt das Problem zu Grunde, ein Verfahren zur Bestimmung von Eigenschaftsänderungen einer einer künstlichen Bewitterung ausgesetzten Probe so weiterzubilden, daß hohe Raffungsfaktoren erzielt werden, um Prüfzeiten für Qualitätskontrollen so kurz wie möglich zu machen. Dabei soll gleichzeitig sichergestellt werden, daß Eigenschaftsänderungen durch unerwünschte Temperaturerhöhung der Probe durch die die Probe beaufschlagende Strahlung ausgeschlossen werden oder die Erwärmung der Proben unabhängig von der photochemisch wirksamen Strahlung zum Beispiel durch eine zusätzliche IR-Strahlung definiert eingestellt werden kann.

Erfindungsgemäß wird das Problem durch die dem Anspruch 1 zu entnehmenden Maßnahmen gelöst.

In grundsätzlicher Abweichung vom vorbekannten Stand der Technik wird die Eigenschaftsänderung der Probe während der künstlichen Bewitterung durch laufende Messung der durchgelassenen oder reflektierten Strahlung zum Beispiel mittels eines UV-Spektralradiometers verfolgt. Dabei ist das erfindungsgemäße Verfahren insbesondere für transparente oder semitransparente Proben geeignet, d.h. direkt anwendbar für Folien, freie Lackfilme oder transparente Decklacke. Bei undurchlässigen Proben kann die Änderung der optischen Eigenschaften auch in Reflektion gemessen werden. Alternativ und als hervorzuhebende Weiterbildung könnten von undurchlässigen Materialien dünne, noch hinreichend transparente Proben präpariert werden.

Die Erfindung sieht vor, daß die Probe mit einer Strahlung in einem Wellenlängenbereich zwischen 295 und 320 nm bestrahlt wird. Dabei kann die Probe mit einer Bestrahlungsstärke bestrahlt werden, die in etwa dem zehnfachen der Sonnenstrahlung in dem entsprechenden Spektralbereich entspricht, ohne daß eine merkliche Temperaturerhöhung der Probe selbst erfolgt. Dabei sollten sowohl der Spektralbereich als auch die Bestrahlungsstärke derart eingestellt werden, daß die Probe mit einer Strahlung im UV-Wellenlängenbereich und einer Stärke derart bestrahlt wird, daß die Probe während der Bestrahlung weniger als 3° C erwärmt wird. Alternativ kann der die Sonnenstrahlung simulierende Spektralbereich bis 400 nm ausgedehnt werden. Infolge des erweiterten Spektralbereichs beträgt die Erwärmung eines schwarzen Körpers dann ca. 10° C.

Mit der erfindungsgemäßen Lehre, insbesondere in Kombination mit der erfindungsgemäßen laufenden spektralen Messung der durch die Probe hindurchgehenden UV-Strahlung, die eine frühzeitige Erkennung von Eigenschaftsänderungen der Probe ermöglicht, sind unerwartet hohe Raffungsfaktoren erzielbar, die eine überaus schnelle und gleichzeitig präzise Qualitätskontrolle ermöglichen. Verfälschende Einflüsse durch insbesondere Temperaturerhöhung der zu überprüfenden Probe sind ausgeschlossen. Gleichzeitig wird die Probe mit einer Strahlung in einem Spektralbereich beaufschlagt, die der Sonnenstrahlung entspricht, die die Eigenschaftsänderungen der zu überprüfenden Materialien üblicherweise hervorrufen.

Gegebenenfalls kann die Probe mit visuellem oder IR-Licht beaufschlagt werden, um zusätzlich berührungslos eine definierte Temperatur auf- bzw. in der Probe einstellen zu können.

Weitere Einzelheiten, Vorteile und Merkmale der Erfindung ergeben sich nicht nur aus den Ansprüchen, sondern auch aus der nachfolgenden Beschreibung von der Zeichnung zu entnehmenden Ausführungsbeispielen.

Es zeigen:
- Fig. 1: eine Prinzipdarstellung einer Ultraschnellbewitterungsanordnung,
- Fig. 2: eine spektrale Transmission einer Probe nach unterschiedlichen Bestrahlungsdauern und
- Fig. 3: eine Anordnung zur Ultraschnellbewitterung einer Probe.

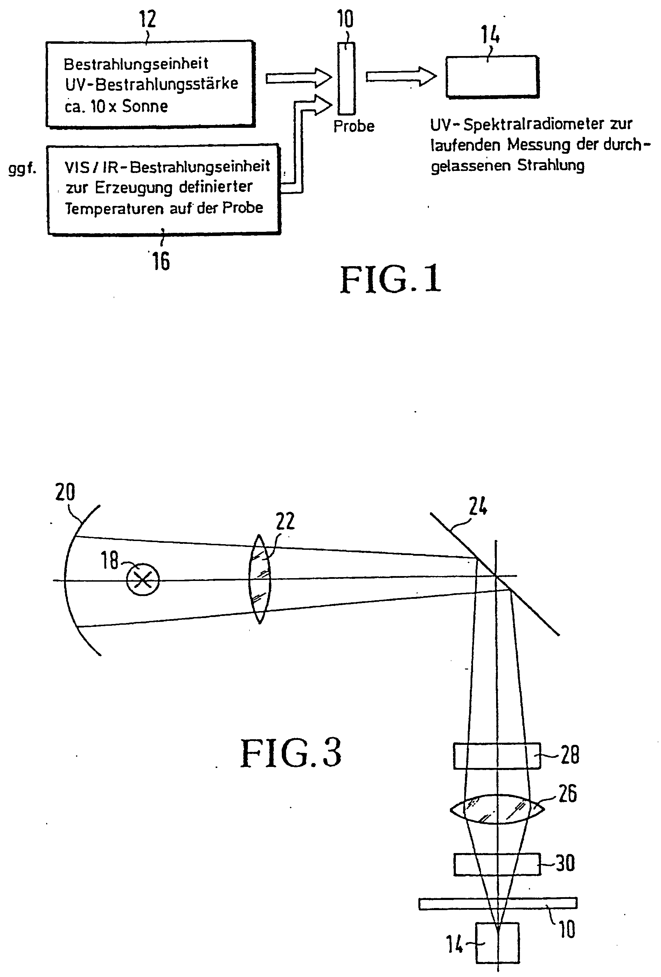

In Fig. 1 ist rein prinzipiell eine Anordnung zur Ultraschnellbewitterung einer Probe 10 in Form eines polymeren Werkstoffs dargestellt. Die Probe 10, bei der es sich um eine transparente, gegebenenfalls um eine semi-transparente Probe handelt, wird mittels einer Bestrahlungseinheit 12 bestrahlt. Die Spektralverteilung des Lichts der Bestrahlungseinheit 12 liegt zwischen 295 ≤ λ ≤ 400 nm, vorzugsweise zwischen 295 ≤ λ ≤ 320 nm, und entspricht der Sonnenstrahlung in diesem Bereich, wobei die Bestrahlungsstärke in diesem Wellenlängenbereich jedoch ca. zehnmal so groß ist wie die der Sonne.

Hinter der Probe 10 ist ein UV-Spektralradiometer 14 zur laufenden Messung der durchgelassenen Strahlung angeordnet. Selbstverständlich kann anstelle des UV-Spektralradiometers 14 auch ein anderes geeignetes Instrument verwendet werden, um die spektrale Transmission τ(λ) der Probe 10 zu ermitteln, um Eigenschaftsänderungen des Probenmaterials in überaus kurzer Zeit zu charakterisieren.

Sofern die Probe 10 auf eine gewünschte Temperatur eingestellt werden soll, kann des weiteren eine visuelle oder IR-Bestrahlungseinheit 16 vorgesehen sein, mittels der eine definierte Temperatur auf der Probe 10 erzeugbar ist.

Eine für die Praxis geeignete Anordnung zur Ultraschnellbewitterung von insbesondere polymeren Werkstoffen, die transparent oder zumindest semitransparent sein sollten, um anhand der durchgelassenen Strahlung Eigenschaftsänderungen während der Bestrahlung ermitteln zu können, ist der Fig. 3 zu entnehmen. Als Strahlungsquelle kann eine Xenonlampe 18 zum Beispiel einer Leistung von 150 W benutzt werden. Die Xenonlampe 18 ist vor einem Kugelreflektor 20 angeordnet. Die von der Xenonquelle 18 unmittelbar bzw. von dem Kugelreflektor 20 stammende Strahlung fällt über ein erstes optisches System 22 auf ein selektiv reflektierendes Filter 24, von dem die Strahlung über ein zweites optisches System 26 auf die Probe 10 gelangt, hinter der das UV-Spektralradiometer 14 angeordnet ist. Das zweite optische System 26 ist derart ausgelegt, daß auf der Probe 10 ein Strahl mit einem Durchmesser von in etwa 10 mm abgebildet wird. Dem zweiten optischen System 26 sind des weiteren Filter 28, 30 vor- und/oder nachgeschaltet, um die kurzwellige Kante der dem Sonnenlicht nachempfundenen Spektralverteilung zu erzeugen.

Durch eine entsprechende Anordnung besteht die Möglichkeit, die Probe 10 über einen Bereich mit einem Durchmesser von zum Beispiel 10 mm mit einer zehnfachen Bestrahlungsstärke im Vergleich zum Sonnenlicht zu beaufschlagen, wobei der Spektralbereich im Wellenlängenbereich zwischen 295 und 320 nm oder zwischen 295 und 400 nm dem des Sonnenlichts entspricht, ohne daß jedoch eine merkliche Erwärmung der Probe 10 selbst erfolgt.

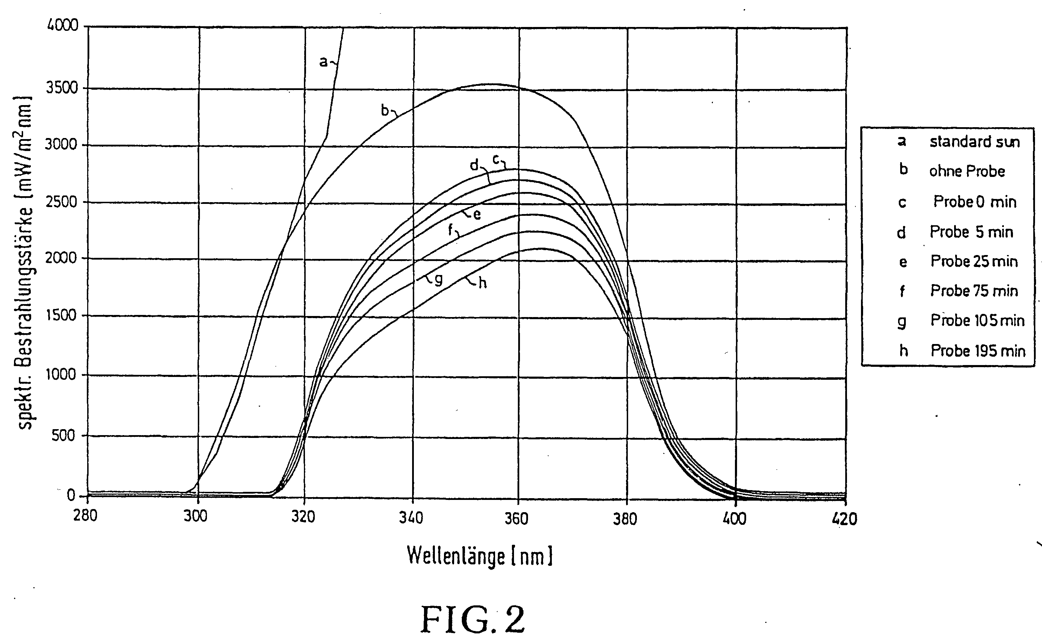

In der Fig. 2 ist die Transmissionsänderung einer PPC-Folie als Probe in Abhängigkeit von der Bestrahlungsdauer wiedergegeben. Dabei ist die von der Probe durchgelassene spektrale Bestrahlungsstärke gegenüber der Wellenlänge aufgetragen.

Wie die Darstellung verdeutlicht, ändert sich sowohbl die Intensität als auch die Spektralverteilung der durchgelassenen Strahlung im Laufe der Bestrahlung. Die Änderung der Transmission τ(λ) des Materials kann folglich mit dem erfindungsgemäßen Verfahren in kürzester Zeit ermittelt werden, bevor visuell eine Veränderung feststellbar ist.

## Patentansprüche

1. Verfahren zur Bestimmung von Eigenschaftsänderungen einer einer künstlichen Bewitterung ausgesetzten Probe, insbesondere einer Probe aus einem polymeren Werkstoff, wobei die Probe in einem Spektralbereich zwischen 295 nm und 400 nm bestrahlt wird, wobei die Spektralverteilung in dem Spektralbereich der Sonnenstrahlung nachempfunden ist und die Bestrahlungsstärke in dem Spektralbereich zumindest fünffach stärker als die der Sonnenstrahlung in dem entsprechenden Spektralbereich ist, und wobei bei gleichbleibinder Bestrohlung die Eigenschaftsänderungen der Probe durch Laufende Messung der spektralen Bestrahlungsstärke der von der Probe durchgelassenen und/oder reflektierten Strahlung als Funktion der Wellenlänge in besagtem Spektralbereich bestimmt wird.

2. Verfahren nach Anspruch 1,
**dadurch gekennzeichnet,**
**daß** die Eigenschaftsänderungen einer Probe bestimmt wird, die transparent oder zumindest semi-transparent ist.

3. Verfahren nach Anspruch 1 oder 2,
**dadurch gekennzeichnet,**
**daß** die Probe mit einer Strahlung im Wellenlängenbereich zwischen in etwa 295 nm und 320 nm bestrahlt wird.

4. Verfahren nach zumindest einem der vorhergehenden Ansprüche,
**dadurch gekennzeichnet,**
**daß** die Probe mit einer Bestrahlungsstärke bestrahlt wird, die in etwa dem zehnfachen der Sonnenstrahlung im Wellenlängenbereich von in etwa 295 nm bis 400 nm entspricht.

5. Verfahren nach zumindest einem der vorhergehenden Ansprüche,
**dadurch gekennzeichnet,**
**daß** die Probe mit einer Strahlung im Wellenlängenbereich von in etwa 295 nm bis 400 nm und einer Bestrahlungsstärke derart bestrahlt wird, daß die Probe weniger als 10° C erwärmt wird.

6. Verfahren nach zumindest einem der vorhergehenden Ansprüche,
**dadurch gekennzeichnet,**
**daß** die Probe mit einer Strahlung im Wellenlängenbereich von in etwa 295 nm bis 320 nm und einer Bestrahlungsstärke derart bestrahlt wird, daß die Probe weniger als 3° C erwärmt wird.

7. Verfahren nach zumindest einem der vorhergehenden Ansprüche,
**dadurch gekennzeichnet,**
**daß** die die Probe beaufschlagende Strahlung auf einen Durchmesser von 5 - 15 mm, vorzugsweise von 10 mm fokussiert wird.

8. Verfahren nach zumindest einem der vorhergehenden Ansprüche,
**dadurch gekennzeichnet,**
**dass** die Probe ergänzend mit IR-Strahlung zur Einstellung der Probe auf eine gewünschte Temperatur beaufschlagt wird.

## Claims

1. A method for determining variations in the properties of a sample exposed to artificial weathering, in particular of a sample consisting of a polymer material, with said sample being irradiated in a spectral range between 295 nm and 400 nm, whereby the spectral distribution in said spectral range simulates solar radiation and the radiation intensity in said spectral range is at least five times greater than that of the solar radiation in the corresponding spectral range, and whereby with constant irradiation the variations in the properties of the sample as function of the wavelength in said spectral range are determined by continuous measurement of the spectral irradiation intensity of the radiation passing through and/or reflected by the sample.

2. Method according to Claim 1,
**wherein**
the variations in the properties are determined of a sample that is transparent or at least semi-transparent.

3. Method according to Claim 1 or Claim 2,
**wherein**
the sample is irradiated with a radiation in the wavelength range between approx. 295 nm and 320 nm.

4. Method according to at least on of the preceding claims,
**wherein**
the sample is irradiated with an irradiation intensity that corresponds to approx. ten times the solar radiation in the wavelength range from approx. 295 nm to 400 nm.

5. Method according to at least on of the preceding claims,
**wherein**
the sample is irradiated with a radiation in the wavelength range from approx. 295 nm to 400 nm and with an irradiation intensity such that the sample is heated less than 10°C.

6. Method according to at least on of the preceding claims,
**wherein**
the sample is irradiated with a radiation in the wavelength range from approx. 295 nm to 320 nm and with an irradiation intensity such that the sample is heated less than 3°C.

7. Method according to at least on of the preceding claims,
**wherein**
the radiation impinging on the sample is focused in a diameter of 5 - 15 mm, preferably of 10 mm.

8. Method according to at least on of the preceding claims,
**wherein**
the sample is impinged supplementary with IR radiation for adjustment of the sample to a required temperature.

## Revendications

1. Procédé pour déterminer les modifications des propriétés d'un échantillon exposé à des intempéries artificielles notamment d'un échantillon d'un polymère, selon lequel
on irradie l'échantillon dans une plage spectrale comprise entre 295 nm et 400 nm, la répartition spectrale dans la plage spectrale étant proche de celle du rayonnement solaire et l'intensité du rayonnement dans la plage spectrale étant au moins cinq fois plus forte que celle du rayonnement solaire dans la plage spectrale correspondante, et
pour un rayonnement restant identique, on détermine les modifications de propriétés de l'échantillon par la mesure continue de l'intensité du rayonnement spectral ayant traversé l'échantillon et/ou réfléchi par l'échantillon en fonction de la longueur d'onde dans la plage spectrale indiquée.

2. Procédé selon la revendication 1,
**caractérisé en ce qu'**
on détermine les modifications de propriétés d'un échantillon qui est transparent ou au moins semi-transparent.

3. Procédé selon la revendication 1 ou 2,
**caractérisé en ce qu'**
on irradie l'échantillon avec un rayonnement compris dans une plage de longueur d'ondes entre environ 295 nm et 320 nm.

4. Procédé selon au moins l'une des revendications précédentes,
**caractérisé en ce qu'**
on irradie l'échantillon avec une intensité de rayonnement qui correspond sensiblement à dix fois celle du rayonnement solaire dans la plage des longueurs d'ondes comprise entre environ 295 nm et 400 nm.

5. Procédé selon au moins l'une des revendications précédentes,
**caractérisé en ce qu'**
on irradie l'échantillon avec un rayonnement dans une plage de longueur d'ondes d'environ 295 nm jusqu'à 400 nm et une intensité de rayonnement qui chauffe l'échantillon de moins de 10°C.

6. Procédé selon au moins l'une des revendications précédentes,
**caractérisé en ce que**
l'échantillon est irradié avec un rayonnement dans une plage de longueur d'ondes comprise entre environ 295 nm et 320 nm et une intensité de rayonnement telle que l'échantillon s'échauffe de moins de 3°C.

7. Procédé selon au moins l'une des revendications précédentes,
**caractérisé en ce que**
le rayonnement appliqué à l'échantillon est focalisé sur un diamètre de 5-15 mm et de préférence 10 mm.

8. Procédé selon au moins l'une des revendications précédentes,
**caractérisé en ce qu'**
on applique à l'échantillon en plus un rayonnement infrarouge pour mettre l'échantillon à une température déterminée.
